# EUROPÄISCHE PATENTSCHRIFT

(11) **EP 0 737 853 B1**
(45) Veröffentlichungstag und Bekanntmachung des Hinweises auf die Patenterteilung: **04.12.2002**
(21) Anmeldenummer: 96103842.9
(22) Anmeldetag: 12.03.1996
(51) Int. Cl.: G01N 11/16, G01N 33/543

(54) **Biosensor**
Biosensor
Capteur biologique

(30) Priorität: 10.04.1995 DE 19512710
(43) Veröffentlichungstag der Anmeldung: 16.10.1996
(73) Patentinhaber: Dade Behring Marburg GmbH, 35001 Marburg (DE)
(72) Erfinder: Meller, Paul, Dr., D-63225 Langen (DE); Madry, Norbert, Dr., D-35041 Marburg (DE); Schelp, Carsten, Dr., D-35041 Marburg (DE); Grzegorzewski, Andrzej, Dr., D-28832 Achim (DE)

(56) Entgegenhaltungen:
- EP-A- 0 177 858
- EP-A- 0 295 965
- EP-A- 0 453 224
- WO-A-89/09937
- US-A- 4 236 893
- US-A- 4 735 906
- US-A- 5 211 054
- ANALYTICAL CHEMISTRY, vol. 66, no. 13, July 1, 1994 Z.A. SHANA et al. "Quartz Crystal Resonators as Sensors in Liquids Using the Acoustoelectric Effect" pages 1955-1964
- ANALYTICAL CHEMISTRY, vol. 64, no. 24, December 15, 1992 MAKOTO MURATSUGU "Detection of Antisteptolysin O Anti- body: Application of an Initial Rate Method of Latex Piezoelectric Immunoassay" pages 2483-2487
- BIOSENSORS & BIOELECTRONICS, vol. 6, 1991 H. MURAMATSU et al. "A quarz viscosity sensor for monitoring coagulation re- action and its application to a multichannel coagulation detector" pages 353-358

## Beschreibung

Die Erfindung betrifft einen Biosensor mit einem Piezoelement als Schwingkreis, von dem eine Meßoberfläche mit Bestandteilen eines zu untersuchenden Fluids unter Reaktion mit testnotwendigen Reagenzien benetzt wird, wobei Viskositäts- und/oder Dichteänderungen des zu untersuchenden Fluids und/oder Masseänderungen durch Ablagerungen an der Meßoberfläche des Piezoelements zu Änderungen der Schwingkreisparameter führen und durch eine elektronische Auswerteschaltung ausgewertet werden.

Allgemeine Ausführungen zu Quarzkristall-Resonatoren sind enthalten in dem Aufsatz von Zack A. Shana und Fabien Josse in Anal. Chem. 1994, 66, Seiten 1955 - 1964, Titel "Quartz Crystal Resonators as Sensors in Liquids Using the Acoustoelectric Effect".

In der EP-A-0 177 858 wird eine Anordnung mit einem Biosensor beschrieben, mit der die Gerinnungszeit von Blut gemessen werden soll. In einer mit einem Deckel verschließbaren Meßkammer ist ein Quarzkristall angeordnet, der als Resonanzkreis an einen Oszillator mit fester Frequenz angeschlossen ist. Das zu untersuchende Blut wird vor Einbringen in die Meßkammer mit für die Gerinnung notwendigen Reagenzien vermischt und dann in die Meßkammer eingebracht. Anschließend wird die Gerinnungszeit dadurch gemessen, daß eine Amplitudenabnahme am Quarzkristall aufgrund der Dämpfung oder Verstimmung des Resonanzkreises durch das gerinnende Blut ausgewertet wird. Die bis zur Gerinnung verstreichende Zeit wird durch eine elektronische Stoppuhr gemessen. Dieser bekannte Biosensor hat den Nachteil, daß nach einem erfolgten Meßvorgang seine Meßkammer bzw. die Meßoberfläche des Quarzkristalls nur sehr schwer zu reinigen ist, und zum anderen die vom Zeitpunkt des Vermischens des Blutes mit für die Gerinnung notwendigen Reagenzien verstreichende Zeit bis zum Aufbringen auf die Meßoberfläche sehr sorgfältig berücksichtigt und kontrolliert werden muß, um Fehlmessungen zu vermeiden.

In dem Aufsatz "A quartz crystal viscosity sensor for monitoring coagulation reaction and its application to a multichannel coagulation detector" von Muramatsu et al in Biosensors & Bioelectronics 6 (1991) Seiten 353 - 358 wird ein Gerät zur Bestimmung der Blutgerinnung beschrieben, bei dem die testspezifischen Komponenten gemischt, bei einer vorgegebenen Temperatur vorinkubiert und dieses Gemisch auf die massensensitive Testfläche des Quarzkristalls gegeben werden muß. Nachteilig wirkt sich bei diesem Verfahren aus, daß die Einflüsse der manuellen Handhabung zu Ungenauigkeiten führen können.

Darüber hinaus sind Biosensoren zum Erkennen von Antigen-Antikörper-Reaktionen beschrieben, die mit Piezoelementen, z.B. einem Quarzkristall, arbeiten. Bei solchen Biosensoren ist auf der Meßoberfläche des Piezoelementes ein Antigen-Antikörper aufgebracht, und das Piezoelement wird anschließend in das zu untersuchende Fluid eingetaucht, um die dabei auftretende Verstimmung des Piezoelementes als Schwingkreis zu messen und auszuwerten. Solche Biosensoren sind z.B. in den US-Patentschriften 4,236,893 und 4,735,906 beschrieben. Doch diese beschriebenen Biosensoren mit einer Immunkomponente auf der Meßoberfläche haben den Nachteil, daß die Meßoberfläche des Piezoelementes bzw. Quarzes bereits durch die Beschichtung modifiziert ist, so daß eine Messung der Schwingfrequenz des Piezoelementes im unbelasteten Zustand nicht mehr möglich ist, was bei Messungen, bei denen das Piezoelement als massensensitives Element eingesetzt wird, sehr nachteilig ist.

Zu der Gruppe solcher Biosensoren, bei den biologische Komponenten aufgebracht sind, gehören auch folgende:

Die EP-A-0 494 896 beschreibt Immunoassays unter Verwendung von modifizierten Quarzkristallmikrowaagen mit Piezokristallen. Diese werden modifiziert, indem biologische Komponenten auf der Kristalloberfläche oder auf einer Polymerzwischenschicht aufgebracht werden. Die Detektion erfolgt über einen enzymatischen Verstärkermechanismus, der ein Produkt erzeugt, das auf der Kristalloberfläche eine Masseänderung durch Adsorption oder durch eine Reaktion mit der Polymerzwischenschicht hervorruft. Die EP-A-0 408 578 beschreibt Immunoassays, bei denen ein Analyt-Einfangreagenz auf einer Trägeroberfläche gebunden ist, die durch einen Abstandshalter über der massensensitiven Fläche fixiert ist. Die Detektion erfolgt dann wie in EP-A-0 494 896 beschrieben. In der EP-A-0 295 965 werden Immunoassay-Verfahren beschrieben, bei denen partikelverstärkte Tests durchgeführt werden. Auf einer modifizierten Sensoroberfläche, auf der Bindungspartner immobilisiert worden sind, werden die partikelverstärkten Immunkomplexe nach einer Immunreaktion durch Trocknen oder Anlegen eines Magnetfeldes bei magnetischen Partikeln auf der Sensoroberfläche verdichtet.

Der Aufsatz "Dectection of Antistreptolysin O Antibody: Application of an Initial Rate Method of Latex Piezoelectric Immunoassay" von Muratsugu et al. in Anal. Chem. 1992, 46, Seiten 2483 - 2487 beschreibt ein Verfahren unter dem Namen "Latex piezoelektrischer Immunoassay", bei dem anstelle der sonst üblichen visuellen Detektion der Latex-Agglutination über turbidimetrische und nephelometrische Verfahren ein Piezokristall genutzt wird, der die Viskositäts- bzw. Dichteänderung der Reaktionslösung erfaßt.

Nachteilig bei den aufgeführten Anordnungen ist, daß die notwendigen Sensitivitäten und Detektionsgrenzen nur durch Modifikationen der Sensoroberfläche und Verstärkermechanismen erreicht werden können. Außerdem ist die Handhabung komplex und aufwendig. Zur Anwendung als Immunoassays sind mehrere Handgriffe notwendig, die zu Ungenauigkeiten führen können. Gerade für die in der Gerinnungsdiagnostik eingesetzten Piezo-Aufbauten führen die sehr komplexen Handgriffe (Mischen der Reagenzien und Vorinkubation, Applikation der Probe) zu Ungenauigkeiten in der Bestimmung der Gerinnungszeit der Probe.

Der vorliegenden Erfindung liegt die Aufgabe zugrunde, einen Biosensor mit einem Piezoelement als Schwingkreis zu schaffen, der Viskositäts- und/oder Dichteänderungen eines zu untersuchenden Fluids und/oder Masseänderungen durch Ablagerungen auf dem Piezoelement ermöglicht, einfach aufgebaut ist, keine komplexen Durchführungsschritte bei der Handhabung erfordert und kein vorheriges Mischen von Reaktionskomponenten mit der zu messenden Probe erforderlich macht und zu einer höheren Meßgenauigkeit führt.

Diese Aufgabe wird gemäß der vorliegenden Erfindung mit einem Biosensor entsprechend dem Oberbegriff des Anspruches 1 dadurch gelöst, daß der Biosensor mit einer gerinnungauslösenden Oberfläche ausgestattet ist, wobei diese Oberfläche
a) ein Trägerkörper, der die testnotwendigen Reagenzien enthält,
b) eine gerinnungsauslösende Beschichtung oder
c) der Trägerkörper an sich die Oberfläche als solche sein kann, und
d) das zu untersuchende Fluid auf den Trägerkörper aufbringbar ist und mit den Reagenzien und der Meßoberfläche in Berührung kommt.

Eine vorteilhafte Ausführungsform des erfindungsgemäßen Biosensors ist dadurch gekennzeichnet, daß der Trägerkörper auf der der Meßoberfläche abgewandten Seite mit einer Filterschicht zum Abtrennen von den Test störenden Substanzen versehen ist, oder der Trägerkörper ist auf der der Meßoberfläche abgewandten Seite mit einer Schicht zur Probenkonditionierung versehen. Hierdurch können evtl. störende Schwebstoffe in dem zu untersuchenden Fluid zurückgehalten werden oder das Fluid kann für den Test konditioniert, z.B. der pH-Wert eingestellt werden.

Eine Variante sieht vor, daß entweder die Sensoroberfläche ohne zusätzliche Beschichtung als gerinungsaktivierendes Agenz eingesetzt wird oder der Trägerkörper, ohne daß er gerinnungsaktivierende Reagenzien enthält, selbst schon gerinnungaktivierend wirkt, und daß das zu untersuchende Fluid auf den Trägerkörper aufbringbar ist und mit den Agenzien sowie der Meßoberfläche in Berührung kommt.

Weitere vorteilhafte Ausführungsformen sind in den Ansprüchen beschrieben.

Hiermit wurde ein Biosensor gefunden, mit dem sozusagen "Trockentests" durchgeführt werden können, d.h., das zu untersuchende Fluid kann in den Biosensor für den Test eingebracht werden, ohne daß zusätzliche Reagenzien notwendig sind, da alle Reagenzkomponenten oder Agenzien bereits in dem Biosensor enthalten sind. Diese testnotwendigen, bereits vorhandenen Reagenzien in dem Biosensor belasten auch nicht die Meßoberfläche des Piezoelementes, so daß genaue Messungen bzw. Tests möglich sind.

Der erfindungsgemäße Biosensor ist vorzugsweise als Einwegteil ausgebildet, was aufgrund des einfachen Aufbaus und der damit verbundenen niedrigen Kosten möglich und wirtschaftlich ist. Vorzugsweise ist der Biosensor zur Bestimmung von Parametern des Blutgerinnungssystems einsetzbar; er kann jedoch mit großem Vorteil auch als Immunoassay verwendet werden.

Bei der Verwendung von gerinnungaktivierenden Trägerkörpern besteht das Material vorteilhaft aus Glaskugeln, - partikeln, -stäuben, -fasern, -vliesen oder ähnlichen partikulären Systemen, die direkt auf der Sensoroberfläche oder in dem Meßraum in Form eines dünnen Films oder lose aufgebracht sind. Es können auch andere dem Fachmann an sich bekannte gerinnungsaktivierende Substanzen genutzt werden, wie z. B. Kaoline, Feldspate, Silicate oder andere aktivierende Körper, die eine negative Oberflächenladung tragen. Bei einer solchen Lösung ist es unschädlich, daß die Körper auf der Meßoberfläche des Piezoelementes aufliegen; diese Berührung führt überraschenderweise nicht zu einer nennenswerten Vorbelastung des Piezoelementes, so daß die Schwingungsparameter auch vor dem Einfüllen des zu untersuchenden Fluids recht genau gemessen werden können.

Bei der Verwendung des Trägerkörpers für die testnotwendigen Reagenzien besteht der Trägerkörper vorzugsweise aus Vlies oder Papier; eine andere vorteilhafte Lösung besteht auch darin, daß der Trägerkörper aus einem Kunststoffgewebe- oder gewirke besteht.

Eine weitere vorteilhafte Ausführungsform des erfindungsgemäßen Biosensors ist dadurch gekennzeichnet, daß die gerinnungsaktiven Agenzien auf der der Meßoberfläche abgewandten Seite mit einer Filterschicht zum Abtrennen von den Test störenden Substanzen versehen ist, oder der Trägerkörper ist auf der der Meßoberfläche abgewandten Seite mit einer Schicht zur Probenkonditionierung versehen. Hierdurch können evtl. störende Schwebstoffe in dem zu untersuchenden Fluid zurückgehalten werden oder das Fluid kann für den Test konditioniert, z.B. der pH-Wert eingestellt werden.

Wird der erfindungsgemäße Biosensor als Blutgerinnungssensor benutzt, so enthält der Trägerkörper gerinnungsauslösende Reagenzien oder das Piezoelement wird selbst als gerinnungsauslösendes Agenz eingesetzt. Eine weitere Variante des erfindungsgemäßen Biosensors als Blutgerinnungssensor sieht vor, daß anstatt eines reagenzientragenden Trägerkörpers eine gerinnungsauslösende Substanz auf das Piezoelement aufgelegt bzw. aufgezogen wird.

Im Falle der Benutzung als Immunoassay enthält der Trägerkörper Immunkomponenten als Reagenzien. Die Immunkomponenten sind vorteilhafterweise an Partikel gebunden, die der Fachmann an sich aus nephelometrischen bzw. turbidimetrischen Verfahren kennt. Diese Partikel können analog zu den gerinnungsauslösenden Agenzien auch direkt auf die Meßoberfläche aufgelegt oder in einem leicht wasserlöslichen Film in der Meßkammer aufgezogen werden.

Eine bevorzugte Meßanordnung mit einem Biosensor gemäß der Erfindung ist dadurch gekennzeichnet, daß eine Oszillatorschaltung sowie eine Mikroprozessorschaltung als elektronische Auswerteschaltung vorgesehen sind, daß das Piezoelement als frequenzbestimmendes Element in die Oszillatorschaltung eingefügt ist und die Frequenzänderungen digital durch die Mikroprozessorschaltung gemessen und ausgewertet werden. Die Mikroprozessorschaltung kann in einem solchen Fall die Zeitkomponente bei der Messung berücksichtigen und durch entsprechende Programmierung eine qualitative und/oder quantitative Beurteilung vornehmen. Um zu besonders stabilen Messungen zu kommen, ist der Biosensor vorzugsweise mit einer Einrichtung zum Temperieren versehen, um die Temperatur auf einem konstanten Wert zu halten. Mit einer Meßanordnung mit Mikroprozessorschaltung ist es möglich, die Temperaturabhängigkeit des Piezoelementes zum Messen der Temperatur im Biosensor auszunutzen, um davon abhängig die Auswertung zu steuern oder ein Temperieren des Biosensors auf eine konstante Temperatur durchzuführen.

Weitere vorteilhafte Ausgestaltungen der Erfindung sind den Unteransprüchen zu entnehmen.

Die Erfindung wird nachfolgend anhand von Ausführungsbeispielen unter Bezug auf die beigefügten Zeichnungen näher erläutert.

Es zeigen:
- Figur 1: einen Querschnitt durch einen Biosensor gemäß der Erfindung in Sandwichbauweise;
- Figur 2: eine Draufsicht auf eine das Piezoelement tragende Schicht des Biosensors nach Figur 1 und
- Figur 3: ein Blockschaltbild einer Meßanordnung mit dem erfindungsgemäßen Biosensor.

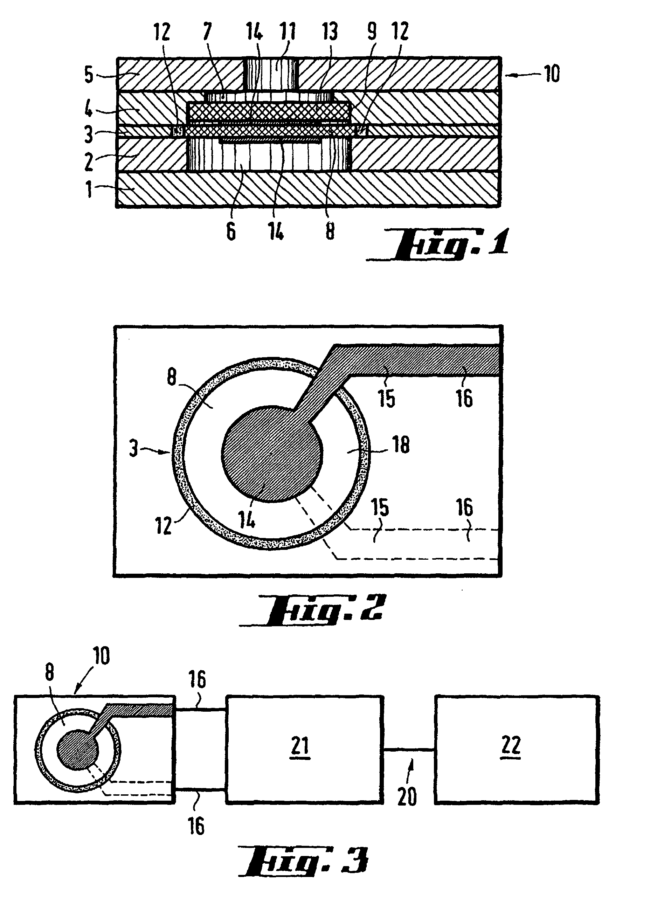

Der in Figur 1 gezeigte Biosensor in Sandwichbauweise enthält fünf Schichten. Eine erste Schicht 1 bildet den Boden des Biosensors 10, eine zweite Schicht 2 schafft durch eine entsprechende Aussparung einen Freiraum 6 unter einem Piezoelement 8, eine dritte Schicht 3 dient als Träger für das Piezoelement 8, eine vierte Schicht bildet durch eine entsprechende Aussparung 9 eine Meßkammer 7 über dem Piezoelement 8 und schließlich bildet eine fünfte Schicht 5 einen Abschluß für die Meßkammer 7 über dem Piezoelement 8, wobei ein Zugang 11 in Form einer Bohrung das Einfüllen des zu untersuchenden Fluids in die Meßkammer 7 zuläßt. Es ist auch möglich, statt der abdeckenden Schicht 5 mit Bohrung 11 eine für das zu untersuchende Fluid durchlässige Membran vorzusehen.

Wie auch aus Figur 2 hervorgeht, ist das Piezoelement 8 auf einer die dritte Schicht 3 bildenden Trägerplatte angeordnet, und zwar ist das Piezoelement 8 mittels eines Klebers 12 in eine entsprechende Aussparung in der dritten Schicht 3 eingeklebt. Beide Flächen des Piezoelementes 8 sind mit Elektroden 14 versehen, die über auf die dritte Schicht 3 aufgebrachte Leiterbahnen 15 mit Anschlußflächen 16 verbunden sind. Die (in Figur 1 gesehen) obere Fläche, in Figur 2 mit dem Bezugszeichen 18 versehen, bildet die Meßoberfläche des Piezoelementes 8, wobei als dichte- bzw. viskositäts- und massensentive Fläche die Fläche der Elektroden 14 zu bezeichnen ist.

In die Meßkammer 7 (siehe Figur 1) des Biosensors 10, also in die Aussparung 9, ist ein Trägerkörper 13 eingesetzt, der lose auf der Meßoberfläche 18 des Piezoelementes 8 aufliegt. Der Trägerkörper 13 dient zur Aufnahme von testnotwendigen Reagenzien, mit denen das zu untersuchende Fluid reagieren soll. Beim Einfüllen des zu untersuchenden Fluids über den Zugang bzw. die Bohrung 11 reagiert das Fluid mit den im Trägerkörper 13 befindlichen Reagenzien und erreicht, zusammen mit den Reagenzien, die Meßoberfläche 18 des Piezoelementes 8. Dieses bedeutet also, daß der Meßbeginn mit dem Einfüllen des zu untersuchenden Fluids exakt festgelegt wird, und es nicht wie bisher auf die Geschicklichkeit der Bedienungsperson ankommt, wie schnell sie das zu untersuchende Fluid mit den Reagenzien vermischt und dann in den Biosensor einfüllt.

Der in den Figuren 1 und 2 dargestellte Biosensor 10 in Sandwichform kann auf geeignete Weise, z.B. durch Verkleben der einzelnen Schichten 1-5 hergestellt werden. Die einzelnen Schichten 1 bis 5 bestehen vorzugsweise aus Kunststoffolien; es ist jedoch auch die Verwendung von Papier für einige der Schichten möglich. Außerdem ist es möglich, einige der Schichten 1-5 zusammenzufassen, z.B. die Schichten 1 und 2 sowie 4 und 5, wobei die jeweiligen Aussparungen für den Freiraum 6 bzw. die Meßkammer 7 durch Prägen hergestellt werden.

Die Herstellung des Biosensors 10 ist sehr einfach und kostensparend, so daß er als Einweg-Biosensor ausgebildet und eingesetzt werden kann. Die Einwegform hat vor allem den Vorteil, daß der Biosensor 10 fertig montiert und mit den testnotwendigen Reagenzien versehen ausgeliefert werden kann; eine Wiederverwendung wäre bei einem solchen Aufbau nicht sinnvoll.

Die in Figur 3 gezeigte Meßanordnung enthält eine Auswerteschaltung 20, die eine Oszillatorschaltung 21, eine Mikroprozessorschaltung 22 sowie den Biosensor 10 aufweist, dessen Piezoelement 8 über die Anschlußflächen 16 mit der Oszillatorschaltung 21 verbunden ist. Die Oszillatorschaltung 21 benutzt das Piezoelement 8, das zweckmäßigerweise ein Quarzkristall ist, als frequenzbestimmendes Element, während die Mikroprozessorschaltung 22 eine entsprechende Frequenzmeßanordnung zum Messen der Schwingungsfrequenz des Oszillators 21 aufweist. Der Mikroprozessor kann außerdem so programmiert und ausgebildet sein, daß er die Frequenzänderungen oder sonstige Parameter des Piezoelementes 8 bzw. der Oszillatorschaltung 21 auswertet und dabei die Zeitkomponente der entsprechenden Änderungen berücksichtigt. Durch entsprechende Programmierung kann aus diesen Meßwerten eine geeignete Auswertung erfolgen und auf einem Display oder einer anderen Ausgabeeinheit (nicht dargestellt) ausgegeben werden.

Da die Schwingungsparameter, insbesondere die Schwingungsfrequenz, des Piezoelementes 8 temperaturabhängig sind, ist es zweckmäßig, die Temperatur des Piezoelementes auf einem konstanten Wert zu halten. Hierzu ist eine geeignete Temperaturregelschaltung für das Piezoelement vorgesehen, wobei die Temperaturabhängigkeit des Piezoelementes 8 in vorteilhafter Weise zum Messen der Ist-Temperatur ausgenutzt wird. Durch eine geeignete Regelschaltung, die zweckmäßigerweise mit dem vorhandenen Mikroprozessor aufgebaut ist, kann dann eine geeignete Temperaturregelung realisiert werden, die in den Testpausen die Betriebsparameter des Piezoelementes konstant hält. Eine solche Regelschaltung ist nicht im einzelnen gezeigt.

Nachfolgend werden nun einige Beispiele in Verbindung mit Versuchen beschrieben.

### Ausführungsbeispiel im Zusammenhang mit der Bestimmung der Gerinnungszeiten (Prothrombinzeit) von lyophilisiertem, normalen, Humanplasmapool sowie lyophilisierten Kontrollen

Vorimprägnierung: Als Trägermaterial für den Trägerkörper 13 wird ein Papier der Firma Macherey und Nagel (MN 215) verwendet, das in eine 0,5 %ige Gelatine-Lösung getaucht wird. Überschüssige Flüssigkeit wird durch Abrollen durch zwei Metallrollen entfernt, und das Papier wird nachfolgend 60 Minuten bei 50 °C getrocknet. 0,1 M Calciumchlorid-Lösung wird nach dem gleichen Prozedere aufgebracht. Die Trocknung erfolgt dann innerhalb von 30 Minuten bei ebenfalls 50 °C.

Auftragen der Gerinnungsreagenzien: 100 µl Phospholipon 25 P (100 g/L, Natterman) und 100 µl Gewebsfaktor (1 g/L, Behringwerke AG) werden mit 25 µl 20 % Triton X 100 gemischt und 2 Stunden bei -20 °C inkubiert. Diese Lösung wird 1:5 mit einem Imprägnierpuffer (0,1 % HSA, 0,1 % Haemaccel, 0,1 % Thiocid in 20 mM HEPES pH 7,3) verdünnt und auf das Papier aufgetragen, vorzugsweise durch Tränken. Die Trocknung erfolgt dann innerhalb von 20 Minuten bei 37 °C.

Bestimmung der Gerinnungszeit einer Plasmaprobe (Prothrombinzeit) (Quicktest):

Das so gefertigte Papier wird auf eine geeignete Fläche zugeschnitten und direkt auf die Meßoberfläche 18 des Piezoelements 8 aufgelegt. Das Piezoelement 8 wird an eine geeignete elektronische Schaltung zur Anregung und Messung der Eigenschwingfrequenz angeschlossen. Die Schwingfrequenz wird erfaßt, z.B. mit einem Frequenzzähler der Firma Keithley (Modell 775) mit einem Datenanschluß an einen PC zur Datenerfassung.

Zur Bestimmung der Gerinnungszeit nach Quick wurden Standard-Human-Plasma (Ch.-B. 502 546, Behringwerke AG), Pathoplasma I (Ch.-B. 502 876, Behringwerke AG) und II (Ch.-B. 502 969, Behringwerke AG) verwendet. Die Probe (20 µl) wird auf den Trägerkörper 13 gegeben und die Abnahme der Schwingfrequenz über die Zeit mitverfolgt. Aktiviert wird das Gerinnungssystem der Plasmaprobe durch Thromboplastin und Calciumionen. Nach einer Verzögerungsphase macht sich die Auslösung der Gerinnung durch eine Abnahme der Schwingfrequenz des Piezoelements 8 zu niedrigen Werten hin bemerkbar. Anhand des typischen Kurvenverlaufs kann mittels anerkannten Auswerteverfahren die Gerinnungszeit der Proben ermittelt werden.

Bei den Meßreihen wurden für die eingesetzten Plasmaproben unterschiedliche Gerinnungszeiten festgestellt. Die Meßergebnisse sind in der nachfolgenden Tabelle dargestellt:

Bestimmung der Prothrombinzeit der Pathoplasma I Probe mit dem Biosensor 10 im Vergleich zu den angegebenen Sollwerten für Standard-Human-Plasma und Pathoplasma II. (Angegeben sind die Mittelwerte zweier Bestimmungen.)

**Tabelle 1**

| | | Standard-Human-Plasma | Pathoplasma II | Probe: Pathoplasma I |
|---|---|---|---|---|
| Sollwerte | % der Norm | 98 | 13 | 24,3 |
| | Bereich | | 10 - 16 | 21,3 - 27,3 |
| Sensor-Kalibrierung | % der Norm | 98 | 13 | |
| Gefundener Wert | % der Norm | | | 23,6 |

### Ausführungbeispiel in Zusammenhang mit der Bestimmung von Vollblut

Als gerinnungsaktives Agenz für den Trägerkörper wird ein Glasfaservlies der Firma Whatman (GFF) verwendet.

Bestimmung der Gerinnungszeit von Vollblut (Citrat-Vollblut recalzifiziert): Das Vlies wird auf eine geeignete Fläche zugeschnitten und direkt auf die Meßoberfläche 18 des Piezoelementes 8 aufgelegt. Das Piezoelement 8 wird an eine geeignete elektronische Schaltung zur Anregung und Messung der Eigenschwingfrequenz angeschlossen. Die Schwingfrequenz wird erfaßt, z. B. mit einem Frequenzzähler der Firma Keithley (Model 775) mit einem Datenanschluß an einen PC zur Datenerfassung.

In die Meßkammer werden 80 µl Vollblut pipettiert und die Abnahme der Schwingfrequenz über die Zeit mitverfolgt. Aktiviert wird das Gerinnungssystem der Probe durch die gerinnungsaktivierende Oberfläche des Vlieses. Nach einer Verzögerungsphase macht sich die startende Gerinnungskaskade durch eine Abnahme der Schwingfrequenz des Piezoelementes 8 zu niedrigen Werten hin bemerkbar. Anhand des typischen Kurvenverlaufs kann mittels anerkannten Auswerteverfahren die Gerinnungszeit der Proben ermittelt werden.

### Ausführungsbeispiel im Zusammenhang mit der Bestimmung der Gerinnungszeit von Vollblut

Das Piezoelement 8 wird an eine geeignete elektronische Schaltung zur Anregung und Messung der Eigenschwingfrequenz angeschlossen. Die Schwingfrequenz wird erfaßt, z. B. mit einem Frequenzzähler der Firma Keithley (Modell 775) mit einem Datenanschluß an einen PC zur Datenerfassung.

In die Meßkammer, die keine gerinnungsaktivierenden Substanzen oder ein Trägermaterial enthält, werden 80 µl Vollblut ( Citrat-Vollblut recalzifiziert) pipettiert und die Abnahme der Schwingfrequenz über die Zeit mitverfolgt. Aktiviert wird das Gerinnungssystem der Probe durch die gerinnungsaktivierende Oberfläche des Sensors. Nach einer Verzögerungsphase macht sich die startende Gerinnungskaskade durch eine Abnahme der Schwingfrequenz des Piezoelementes 8 zu niedrigen Werten hin bemerkbar. Anhand des typischen Kurvenverlaufs kann mittels anerkannten Auswerteverfahren die Gerinnungszeit der Proben ermittelt werden.

### Ausführungsbeispiel eines Latex-verstärkten Immunoassays zur Bestimmung von Rheumafaktoren (Rf-Test)

Die vorimprägnierten Trägermaterialien werden mit Rf-Latexreagenzien, wie im ersten Beispiel beschrieben, imprägniert und in dem gleichen Aufbau verwendet. Durch Zugabe einer Rf-positiven Probe findet die Agglutinationsreaktion statt, die durch die Abnahme der Schwingfrequenz des Piezoelementes 8 mitverfolgt wird. Die Bestimmung des Rf-Gehaltes geschieht dann entweder über eine Endpunktsbestimmung oder über die Reaktionsgeschwindigkeit, die in Relation zu der zeitlichen Frequenzänderung steht.

## Patentansprüche

1. Biosensor mit einem Piezoelement als Schwingkreis, von dem eine Meßoberfläche mit Bestandteilen eines zu untersuchenden Fluids unter Reaktion mit testnotwendigen Reagenzien benetzt wird, wobei Viskositäts- und/oder Dichteänderungen des zu untersuchenden Fluids und/oder Masseänderungen durch Ablagerungen an der Meßoberfläche des Piezoelements zu Änderungen der Schwingkreisparameter führen und durch eine entsprechende elektronische Auswerteschaltung ausgewertet werden, **dadurch gekennzeichnet, daß** der Biosensor mit einer gerinnungsauslösenden Oberfläche ausgestattet ist, und das zu untersuchende Fluid auf den Trägerkörper (13) aufbringbar ist und gegebenenfalls mit den Reagenzien sowie der Meßoberfläche (18) in Berührung kommt.

2. Biosensor nach Anspruch 1 **dadurch gekennzeichnet, daß** die Sensoroberfläche ohne zusätzliche Beschichtung als gerinnungaktivierendes Agenz eingesetzt wird.

3. Biosensor nach Anspruch 1 **dadurch gekennzeichnet, daß** die testnotwendigen Reagenzien in einem Trägerkörper (13) enthalten sind, der auf die Meßoberfläche (18) des Piezoelementes (8) aufgelegt ist.

4. Biosensor nach Anspruch 1 **dadurch gekennzeichnet, daß** der Trägerkörper (13) selbst, ohne gerinnungsaktivierende Reagenzien zu enthalten, gerinnungsaktivierend wirkt, und direkt auf der Meßoberfläche (18) des Piezoelements aufgelegt wird oder in der Meßkammer (7) aufgebracht wird.

5. Biosensor nach einem der Ansprüche 1-4 **dadurch gekennzeichnet, daß** er als Einwegteil ausgebildet ist.

6. Biosensor nach Anspruch 3 **dadurch gekennzeichnet, daß** der Trägerkörper (13) aus Vlies oder Papier besteht.

7. Biosensor nach Anspruch 3 **dadurch gekennzeichnet, daß** der Trägerkörper (13) aus einem Kunststoffgewebe oder -gewirke besteht.

8. Biosensor nach Anspruch 3 **dadurch gekennzeichnet, daß** der Trägerkörper (13) aus Glaskugeln, -fasern, -partikeln, oder -stäuben besteht.

9. Biosensor nach Anspruch 3 **dadurch gekennzeichnet, daß** der Trägerkörper (13) aus Kaolin, Feldspaten oder Silicat besteht.

10. Biosensor nach Anspruch 3 **dadurch gekennzeichnet, daß** der Trägerkörper (13) aus einer Substanz mit einer negativen Oberflächenladung besteht.

11. Biosensor nach einem der Ansprüche 1 bis 10 **dadurch gekennzeichnet, daß** der Trägerkörper (13) auf der der Meßoberfläche (18) abgewandten Seite mit einer Filterschicht zum Abtrennen von den Test störenden Substanzen versehen ist.

12. Biosensor nach einem der Ansprüche 1 bis 10 **dadurch gekennzeichnet, daß** der Trägerkörper (13) auf der der Meßoberfläche abgewandten Seite mit einer Schicht zur Probenkonditionierung versehen ist.

13. Biosensor nach einem der Ansprüche 3, 11 und 12 zur Benutzung als Blutgerinnungssensor **dadurch gekennzeichnet, daß** der Trägerkörper (13) gerinnungsauslösende Reagenzien enthält.

14. Biosensor nach einem der Ansprüche 3, 11 und 12 zur Benutzung als Immunoassay **dadurch gekennzeichnet, daß** der Trägerkörper (13) Immunkomponenten als Reagenzien enthält.

15. Biosensor nach einem der vorstehenden Ansprüche **dadurch gekennzeichnet, daß** das Piezoelement (8) ein als Scherschwinger ausgebildeter Quarzkristall ist.

16. Biosensor nach einem der Ansprüche 5 bis 15 **dadurch gekennzeichnet, daß** er sandwichartig aus mehreren Schichten (1, 2, 3, 4, 5) zusammengesetzt, insbesondere zusammengeklebt, ist.

17. Biosensor nach Anspruch 16 **dadurch gekennzeichnet, daß** er fünf Schichten (1 - 5) enthält, nämlich
- eine erste, einen geschlossenen Boden bildende Schicht (1)
- eine zweite, einen Freiraum (6) unter dem Piezoelement (8) lassende Schicht (2),
- eine dritte, das Piezoelement (8) haltemde Schicht (3),
- eine vierte, die Meßkammer (7) über dem Piezoelement (8) bildende Schicht (4), die gleichzeitig den Trägerkörper (13) haltert, und
- eine fünfte, die Meßkammer (7) über dem Piezoelement (8) als Deckel abschließende Schicht (5) mit einem Zugang (11) zum Einfüllen des zu untersuchenden Fluids in die Meßkammer (7).

18. Biosensor nach einem der Ansprüche 1 bis 17 **dadurch gekennzeichnet, daß** der Zugang zum Einbringen des zu untersuchenden Fluids aus einer mit der Meßkammer (7) in Verbindung stehenden Bohrung (11) besteht.

19. Biosensor nach einem der Ansprüche 1 bis 14 **dadurch gekennzeichnet, daß** der Zugang zum Einbringen des zu untersuchenden Fluids aus einer die Meßkammer (7) abdeckenden, für das Fluid durchlässigen Membran besteht.

20. Meßanordnung mit einem Biosensor nach einem der vorstehenden Ansprüche **dadurch gekennzeichnet, daß** sie eine Oszillatorschaltung (21) und eine Mikroprozessorschaltung (22) als elektronische Auswerteschaltung (20) enthält, daß das Piezoelement (8) als frequenzbestimmendes Element in die Oszillatorschaltung (21) eingefügt ist und daß Frequenzänderungen digital durch die Mikroprozessorschaltung (22) gemessen und ausgewertet werden.

21. Biosensor nach einem der vorstehenden Ansprüche **dadurch gekennzeichnet, daß** er mit einer Einrichtung zum Temperieren versehen ist, um seine Temperatur auf einem konstanten Wert zu halten.

22. Meßanordnung nach Anspruch 20 **dadurch gekennzeichnet, daß** die Temperaturabhängigkeit des Piezoelementes (8) zum Messen der Temperatur ausgenutzt wird.

## Claims

1. A biosensor having a piezoelectric element as resonant circuit, a measurement surface of which is exposed to components of a fluid to be examined by reaction with reagents necessary for the test, viscosity and/or density changes in the fluid to be examined and/or mass changes due to deposition on the measurement surface of the piezoelectric element leading to changes in the resonant circuit parameters and being evaluated by- a corresponding electronic evaluation circuit, wherein the biosensor is equipped with a coagulation-initiating surface and the fluid to be examined can be applied to the support (13) and, if appropriate, comes into contact with the reagents and the measurement surface (18).

2. The biosensor as claimed in claim 1, wherein the sensor surface is used without additional coating as coagulation activator.

3. The biosensor as claimed in claim 1, wherein the reagents necessary for the test are contained in a support (13) which is put onto the measurement surface (18) of the piezoelectric element (8).

4. The biosensor as claimed in claim 1, wherein the support (13) itself has a coagulation activation action, without needing to contain coagulation activator reagents, and is put directly on the measurement surface (18) of the piezoelectric element or is fitted in the measurement chamber (7).

5. The biosensor as claimed in one of claims 1 to 4, wherein it is designed as a single-use article.

6. The bicsensor as claimed in claim 3, wherein the support (13) consists of nonwoven material or paper.

7. The biosensor as claimed in claim 3, wherein the support (13) consists of a synthetic woven or knitted fabric.

8. The biosensor as claimed in claim 3, wherein the support (13) consists of glass spheres, glass fibers, glass particles or glass dust.

9. The biosensor as claimed in claim 3, wherein the support (13) consists of kaolin, feldspars or silicate.

10. The biosensor as claimed in claim 3, wherein the support (13) consists of a substance having a negative surface charge.

11. The biosensor as claimed in one of claims 1 to 10, wherein the support (13) is provided with a filter layer, for the separation of substances that interfere with the test, on the side remote from the measurement surface (18).

12. The biosensor as claimed in one of claims 1 to 10, wherein the support (13) is provided with a sample-conditioning layer on the side remote from the measuring surface.

13. The biosensor as claimed in one of claims 3, 11 and 12 for use as a blood coagulation sensor, wherein the support (13) contains coagulation-initiating reagents.

14. The biosensor as claimed in one of claims 3, 11 and 12 for immunoassay use, wherein the support (13) contains immunocomponents as reagents.

15. The biosensor as claimed in one of the preceding claims, wherein the piezoelectric element (8) is a quartz crystal designed as a shear-mode oscillator.

16. The biosensor as claimed in one of claims 5 to 15, wherein it is composed in sandwich fashion of a plurality of layers (1, 2, 3, 4, 5), in particular bonded together.

17. The biosensor as claimed in claim 16, wherein it contains five layers (1-5), namely
- a first layer (1) forming a closed base,
- a second layer (2) leaving a free space (6) below the piezoelectric element (8),
- a third layer (3) holding the piezoelectric element (8),
- a fourth layer (4) forming the measurement chamber (7) above the piezoelectric element (8) and simultaneously holding the support (13), and
- a fifth layer (5) closing off the measurement chamber (7) above the piezoelectric element (8) as a lid, and having an access hole (11) for filling the measurement chamber (7) with the fluid to be examined.

18. The biosensor as claimed in one of claims 1 to 17, wherein the access hole for introducing the fluid to be examined consists of a bore (11) communicating with the measurement chamber (7).

19. The biosensor as claimed in one of claims 1 to 14, wherein the access hole for introducing the fluid to be examined consists of a membrane that covers the measurement chamber (7) and is permeable to the fluid.

20. A measurement arrangement having a biosensor as claimed in one of the preceding claims, wherein it contains an oscillator circuit (21) and a microprocessor circuit (22) as electronic evaluation circuit (20), wherein the piezoelectric element (8) is inserted as frequency-determining element into the oscillator circuit (21) and wherein frequency changes are digitally measured by the microprocessor circuit (22) and evaluated.

21. The biosensor as claimed in one of the preceding claims, wherein it is provided with a temperature-control device for keeping its temperature at a' constant value.

22. The measurement arrangement as claimed in claim 20, wherein the temperature dependence of the piezoelectric element (8) is used to measure the temperature.

## Revendications

1. Capteur biologique comprenant un élément piézoélectrique formant le circuit oscillant, dont une surface de mesure est mouillée par des composants d'un fluide à examiner par la réaction avec des réactifs nécessaires pour le test, dans lequel les variations de la viscosité et/ou de la densité du fluide à examiner et/ou les variations de la masse dues à des dépôts sur la surface de mesure de l'élément piézoélectrique entraînent des variations des paramètres du circuit oscillant et sont analysées par un circuit d'analyse électronique, **caractérisé en ce que** le capteur biologique est muni d'une surface stimulant la coagulation et le fluide à examiner peut être posé sur le corps de support (13) et, le cas échéant, être mis en contact avec les réactifs et avec la surface de mesure (18).

2. Capteur biologique selon la revendication 1, **caractérisé en ce que** la surface du capteur est utilisée, sans revêtement supplémentaire, comme agent stimulant la coagulation.

3. Capteur biologique selon la revendication 1, **caractérisé en ce que** les réactifs nécessaires pour. le test sont contenus dans un corps de support (13), qui est posé sur la surface de mesure (18) de l'élément piézoélectrique (8).

4. Capteur biologique selon la revendication 1, **caractérisé en ce que** le corps de support (13) lui-même, sans contenir des agents stimulant la coagulation, agit comme un stimulant de la coagulation et est posé directement sur la surface de mesure (18) de l'élément piézoélectrique ou est inséré dans la chambre de mesure (7).

5. Capteur biologique selon une des revendications 1 à 4, **caractérisé en ce que** ledit capteur biologique est conçu sous forme jetable.

6. Capteur biologique selon la revendication 3, **caractérisé en ce que** le corps de support (13) est réalisé en non-tissé ou en papier.

7. Capteur biologique selon la revendication 3, **caractérisé en ce que** le corps de support (13) est réalisé dans un tissu ou un tricot en matière synthétique.

8. Capteur biologique selon la revendication 3, **caractérisé en ce que** le corps de support (13) est formé par des billes de verre, des fibres de verre, des particules de verre ou des poussières de verre.

9. Capteur biologique selon la revendication 3, **caractérisé en ce que** le corps de support (13) est réalisé à partir de kaolin, de feldspath ou de silicate.

10. Capteur biologique selon la revendication 3, **caractérisé en ce que** le corps de support (13) est réalisé dans une substance, dont la surface porte une charge négative.

11. Capteur biologique selon une des revendications 1 à 10, **caractérisé en ce que** le corps de support (13) est muni, sur sa face opposée à la surface de mesure (18), d'une couche filtrante destinée à séparer du test les substances perturbatrices.

12. Capteur biologique selon une des revendications 1 à 10, **caractérisé en ce que** le corps de support (13) est muni, sur sa face opposée à la surface de mesure, d'une couche destinée à conditionner les échantillons.

13. Capteur biologique selon les revendications 3, 11 et 12, **caractérisé en ce que** le corps de support (13) contient des réactifs stimulant la coagulation.

14. Capteur biologique selon les revendications 3, 11 et 12, **caractérisé en ce que** le corps de support (13) contient des réactifs sous forme de complexes immuns.

15. Capteur biologique selon une des revendications précédentes, **caractérisé en ce que** l'élément piézoélectrique (8) est un cristal de quartz conçu sous forme d'oscillateur de cisaillement.

16. Capteur biologique selon une des revendications 5 à 15, **caractérisé en ce que** ledit capteur biologique est conçu selon un mode de construction sandwich à plusieurs couches (1, 2, 3, 4, 5), en particulier assemblées par collage.

17. Capteur biologique selon la revendication 16, **caractérisé en ce qu'**il contient cinq couches (1-5), à savoir :
- une première couche (1), qui forme un fond fermé,
- une deuxième couche (2), laissant un espace libre (6) au-dessous de l'élément piézoélectrique (8),
- une troisième couche (3) contenant l'élément piézoélectrique (8),
- une quatrième couche (4), qui forme la chambre de mesure (7) au-dessus de l'élément piézoélectrique (8) et qui supporte en même temps le corps de support (13), et
- une cinquième couche (5), fermant comme un couvercle la chambre de mesure (7) au-dessus de l'élément piézoélectrique (8) et munie d'un accès (11) pour introduire le fluide à examiner dans la chambre de mesure (7).

18. Capteur biologique selon une des revendications 1 à 17, **caractérisé en ce que** l'accès pour introduire le fluide à examiner est formé par une forure (11) qui communique avec la chambre de mesure (7).

19. Capteur biologique selon une des revendications 1 à 14, **caractérisé en ce que** l'accès pour introduire le fluide à examiner est formé par une membrane perméable au fluide fermant la chambre de mesure (7).

20. Dispositif de mesure comprenant un capteur biologique selon une des revendications précédentes, **caractérisé en ce qu'**il comporte un circuit à oscillateur (21) et un circuit à microprocesseur (22) formant un circuit d'analyse électronique (20), **en ce que** l'élément piézoélectrique (8) est inséré sous forme d'élément déterminant la fréquence dans le circuit à oscillateur (21) et **en ce que** les variations de fréquence sont mesurées et analysées numériquement par le circuit à microprocesseur (22).

21. Capteur biologique selon une des revendications précédentes, **caractérisé en ce qu'**il est muni d'une unité destinée à équilibrer la température, en vue de maintenir sa température à une valeur constante.

22. Dispositif de mesure selon la revendication 20, **caractérisé en ce que** la dépendance de l'élément piézoélectrique (8) avec la température est exploitée pour mesurer la température.
